# EUROPÄISCHE PATENTANMELDUNG

(11) **EP 3 320 831 A1**
(43) Veröffentlichungstag der Anmeldung: **16.05.2018**
(21) Anmeldenummer: 16198313.5
(22) Anmeldetag: 11.11.2016
(51) Int. Cl.: A61B 5/00, A61B 5/0402, A61B 5/0476, A61B 5/08, A61B 5/113

(54) **VERFAHREN UND VORRICHTUNG ZUR WANDLUNG VON KÖRPERSIGNALEN**

(71) Anmelder: Köhl, Markus, 28211 Bremen (DE)
(72) Erfinder: Köhl, Markus, 28211 Bremen (DE)
(74) Vertreter: Zenz Patentanwälte Partnerschaft mbB

(57) **Zusammenfassung**

Ein Verfahren zum Generieren von Schallinformationen in Abhängigkeit von biologischen Aktivitäten eines menschlichen Organismus. Ein erster zeitlicher Signalverlauf wird aus Messungen einer elektrischen Aktivität eines Gehirns des Organismus erzeugt und daraus wird ein erster Datenstrom generiert. Ein zweiter Datenstrom wird aus Messungen einer elektrischen Aktivität eines Herzens und ein dritter Datenstrom wird aus Messungen der Atmung erzeugt.

In Echtzeit wird ein akustisches Signal im auditiv wahrnehmbaren Frequenzspektrum generiert und über Schallwandler ausgegeben, wobei wenigstens ein Parameter des akustischen Signals zeitlich in Abhängigkeit von aktuellen Datenwerten aus dem ersten Datenstrom, ein Parameter zeitlich in Abhängigkeit von aktuellen Datenwerten aus dem zweiten Datenstrom und ein Parameter zeitlich in Abhängigkeit von aktuellen Datenwerten aus dem dritten Datenstrom bestimmt wird. Die Parameterbestimmung erfolgt gemäß Rechenvorschriften oder gespeicherten Zuordnungen aus den Datenwerten, wobei sich die Rechenvorschriften oder gespeicherten Zuordnungen paarweise unterscheiden, so dass die Veränderungen jedes der Datenströme eine zugeordnete hörbare Veränderung des akustischen Signals hervorrufen.

## Beschreibung

Die Erfindung betrifft ein Verfahren und eine Vorrichtung zum Erzeugen von Schallinformationen in Abhängigkeit von biologischen Aktivitäten eines menschlichen Organismus.

Im menschlichen Körper laufen zeitgleich zahlreiche organische Prozesse ab, die bewusste Wahrnehmung solcher Vorgänge ist für Beobachter einer Person und regelmäßig auch für eine Person selbst schwer möglich. In der Medizintechnik gibt es daher zahlreiche Erfassungsgeräte und Erfassungskonzepte, um Biosignale eines Menschen derart zu wandeln, dass sie beobachtbar oder auswertbar werden.

Beispielsweise sind EEG(Elektroenzephalogramm)-Systeme bekannt, um elektrische Aktivitäten menschlicher Gehirne zu erfassen und auszuwerten. Für die elektrischen Aktivitäten des menschlichen Herzens sind EKG(Elektrokardiographie)-Systeme oder auch optische Überwachungssysteme bekannt und die Atmung von Menschen und Patienten kann über einen Temperatursensor (Airflow/ Nasal/Oral-Flowsensor Thermistor) oder einen Atemgurte (Brust- und Bauchgurt) überwacht werden. Zudem werden EDA-Systeme für die Messung des Hautwiderstandes eingesetzt (EDA-Sensor in der Handinnenfläche) oder Sensoren für die Sauerstoffsättigung im Blut ebenfalls per Pulsoximeter (Fingerclip mit Infrarotdioden und -Transistoren, die die Lichtdurchlässigkeit erfassen).

Für den untersuchten Probanden selbst wie auch für ungeschulte Beobachter sind solche Signale und Auswertungen regelmäßig nicht intuitiv erfassbar und ohne medizinische Vorkenntnisse nicht interpretierbar. Auch für geschulte Überwachungspersonen ist die Auswertung der Daten und Signale regelmäßig aufwendig. Eine Beobachtung dieser zeitlich veränderlichen Informationen, insbesondere Informationen mehrerer gleichzeitig überwachter organischer Vorgänge bei einer In-Situ-Überwachung erfordert die volle Aufmerksamkeit und erfasst regelmäßig dennoch nicht die gesamte Informationstiefe.

Aus dem Stand der Technik ist zu verschiedenen Zwecken bekannt, Körpersignale in grafischer Darstellung oder akustische Signale zu wandeln. Beispielsweise beschreibt das Dokument KR 20040070145 (A) ein Verfahren zur Darstellung von Gehirnwellen als Klänge oder Bilder.

Eine Wandlung von mit den menschlichen Sinnen nicht wahrnehmbaren Körpervorgängen in hörbare Signale ist auch verschiedenen anderen Dokumenten aus dem Stand der Technik bekannt, beispielsweise in

The Sonification Handbook, edited by Thomas Hermann, Andy Hunt, John G. Neuhoff; Logos Publishing House, Berlin 2011, 586 pages, 1. edition (11/2011), ISBN 978-3-8325-2819-5;

Baier, G., Hermann T., & Stephani U. (2007). Event-based sonification of EEG rhythms in real time. Clinical Neurophysiology. 1377-1386;

Wallis, I., Ingalls, T., Rikakis, T., Olsen, L., Chen, Y., Xu, W., & Sundaram, H. (2007). Real-time sonification of movement for an immersive stroke rehabilitation environment. Proceedings of the International Conference on Auditory Display, 497-503.

Die bekannten Systeme wählen zwangsläufig einen Aspekt der Vorgänge im menschlichen Organismus aus und bereiten diesen Aspekt auf. Dabei wird nur ein Ausschnitt des aktuellen Zustandes des Probanden erfasst und vermittelt.

Aufgabe der Erfindung ist es, ein Verfahren und eine Vorrichtung zur Verfügung zu stellen, welche möglichst umfassend Vorgänge im menschlichen Organismus aufbereitet und in eine einfache wahrnehmbare Darbietung wandelt.

Diese Aufgabe wird gelöst durch ein Verfahren zum Generieren von Schallinformationen mit den Merkmalen des Patentanspruches 1 sowie eine Vorrichtung zur Ausführung des Verfahrens.

Gemäß dem erfindungsgemäßen Verfahren werden biologische Aktivitäten eines menschlichen Organismus in Schallinformationen gewandelt. Die Erfindung verdeutlicht das Zusammenwirken der unterschiedlichen Vorgänge im menschlichen Organismus durch eine Darstellungsform, bei welcher alle Messparameter in einer Darstellungsform zusammenkommen.

Wissenschaftlicher Hintergrund dabei ist das rhythmische System von Herz-, Atmungsfunktion, sowie dem kognitiven Rhythmus aus der Gehirntätigkeit mit den Frequenzspektren und Aktivitäten. Die Betrachtung des Atem- und Kreislaufsystems als einem rhythmisch arbeitenden Organsystem ist etabliert. Jeder Mensch erlebt den Herzpuls und die Atemzüge unmittelbar und direkt als rhythmische Vorgänge. Die Bedeutung dieser Rhythmen für die gesamte Physiologie des Menschen wird aber noch nicht ausreichend umfassend gewürdigt. Mit der Beobachtungsmöglichkeit des Zusammenspiels der rhythmischen Vorgänge wird ein Verständnis der organismischen Zeitorganisation geschaffen. Beispielsweise tendiert die Relation von Herzpuls und Atmung in der mitternächtlichen Tiefschlafphase zum ganzzahligen Verhältnis 1:4. Die Rhythmen stehen in einem Verhältnis. Ähnliche ganzzahlige Verhältnisse sind auch zwischen der Atmung und der rhythmischen Blutdruckveränderung oder der periodischen Veränderung der peripheren Organdurchblutung festzustellen. Der gesamte Organismus ist durch vielfältige koordinierte Zeitverläufe gekennzeichnet. Diese Zeitkoordination des Organismus bedeutet ein zeitliches Wechselverhältnis und eine funktionale Koordination der rhythmisch verlaufenden Lebensprozesse. Deshalb hat eine zeitliche Beeinflussung des einen Rhythmus eine nachweisbare Auswirkung auf den gesamten rhythmischen Organismus. Gesundheit ist an eine intakte rhythmische Ordnung der Lebensfunktionen gebunden. Störungen dieser Ordnung bei Krankheiten sind vielfältig nachgewiesen.

Der Herzpuls steht in synchroner Koordination mit zahlreichen Rhythmen des Organismus, zum Beispiel mit dem Rhythmus der Blutdruckwellen oder der Organdurchblutung. Andererseits steht Herzpuls in direkter Abhängigkeit von der Atmung. Anders als der Herzpuls kann die Atmung bezüglich Rhythmus und Tiefe willkürlich verändert werden. Gleichzeitig ist die Atmung unmittelbarster Ausdruck unbewusster oder halbbewusster psychischer Zustände (z.B. ein tiefer Seufzer, ein erlösendes Ausatmen, das Anhalten des Atems bei Spannung usw.). Dies hat wiederum Rückwirkungen auf die Physiologie des Kreislaufs.

Gemäß der Erfindung wird zumindest ein erster zeitlicher Signalverlauf aus Messungen einer elektrischen Aktivität eines Gehirns des menschlichen Organismus erzeugt. Aus diesem ersten zeitlichen Signalverlauf wird ein erster Datenstrom generiert. Der Datenstrom kann beispielsweise durch eine AD-Wandlung aus dem Signalverlauf erzeugt werden und stellt eine in Echtzeit gewandelte digitalisierte Signalinformation bereit. Es werden entsprechend Live-Daten verarbeitet und dargestellt. Diese können zur wissenschaftlichen Auswertung aufgezeichnet werden. Die Erfassung der elektrischen Aktivitäten des Gehirns kann mit herkömmlichen Mitteln, insbesondere einem herkömmlichen EEG erfolgen, welches entsprechende Signalausgänge aufweist, die auf einer Mehrzahl von Kanälen verschiedene Aktivitäten des menschlichen Gehirns erfassen (hierzu kann beispielsweise ein 6,12, oder 48, 56 Kanäle EEG eingesetzt werden, mit 16 oder 24 Bit Signalauflösung, Aufzeichnungsrate für jeden Kanal individuell einstellbar von 4/s bis 512/s; optional 4096/s.

Gemäß der Erfindung wird zeitgleich ein zweiter zeitlicher Signalverlauf aus Messungen einer elektrischen Aktivität eines Herzens des menschlichen Organismus erzeugt, wobei aus dem zweiten zeitlichen Signalverlauf ein zweiter Datenstrom generiert wird. Auch der zweite Datenstrom erfasst in Echtzeit die Vorgänge im Organismus, wobei die Erfassung des Signalverlaufs ebenfalls mit herkömmlichen Geräten für die Registrierung elektrischer Aktivitäten des menschlichen Herzens erfasst werden kann, insbesondere EEG-Systeme oder Herzbrustgurte. Die Art der Datengenerierung des Herzschlages ist für das vorliegende Verfahren beliebig, es kann die Herzfrequenz beispielsweise mit einem 3-Kanal EKG Sensor erfasst werden oder mit einem Fingerclip für Pulsoximeter für die Erfassung der Herzfrequenz).

Weiterhin wird simultan ein dritter zeitlicher Signalverlauf aus Messungen der Atmung des menschlichen Organismus erzeugt. Dieser dritte Signalverlauf wird in einen dritten Datenstrom umgewandelt.

Gemäß der Erfindung erfolgt also simultan eine Erfassung der elektrischen Aktivität des Gehirns, der elektrischen Aktivität des Herzens und der Atmung des zugehörigen menschlichen Organismus, wobei sämtliche Messungen an derselben Person und parallel durchgeführt werden. Eine Wandlung in die Datenströme und weitere Verarbeitung erfolgt in Echtzeit. Im Rahmen dieser Anmeldung ist unter dem Begriff der Echtzeitverarbeitung zu verstehen, dass die Verzögerung zur Wandlung und Weiterverarbeitung der Signale bis zur Ausgabe der akustischen Schallinformationen in einer simultan zur Realität ablaufenden Zeit erfolgt. Die Verarbeitung erfolgt also ohne gezielte Verzögerungen oder Dehnungen und Stauchungen auf einheitlichen Zeitskalen. Außerdem ist unter dem Begriff Echtzeit zu verstehen, dass die Verzögerung zwischen der Erfassung der Daten und deren Verarbeitung und Ausgabe möglichst gering sein soll, die Reaktionszeit also kurz ist. Im Rahmen der Erfindung sollte zwischen der Erfassung der Information und der Ausgabe der Schallinformation entsprechend höchstens eine Zeitdauer von einigen Sekunden, vorzugsweise weniger als eine Sekunde vergehen. Manche EEG-Phänomene lassen sich auch nur bei geringer Zeitverzögerung erkennen. Die Messgeräte erfassen die Werte regelmäßig innerhalb einiger Millisekunden, die akustische Umwandlung durch die Schallwellen liegt ebenfalls im Millisekundenbereich.

Gemäß der Erfindung liegen damit wenigstens drei Datenströme vor, die aus den elektrischen Aktivitäten des Hirns, des Herzens und außerdem aus der Atmung abgeleitet wurden. Im Rahmen der Erfindung ist es durchaus möglich, dass zu jeder dieser organischen Aktivitäten mehrere Signalverläufe und Datenströme generiert werden, es muss jedoch wenigstens ein Datenstrom zu jeder dieser körperlichen Aktivitäten generiert werden.

Aus diesen Datenströmen wird in Echtzeit ein akustisches Signal im auditiv wahrnehmbaren Frequenzspektrum erzeugt und über Schallwandler ausgegeben. Unter Signalen im auditiv wahrnehmbaren Frequenzspektrum sind in diesem Zusammenhang alle für den Menschen hörbaren Signale zu verstehen, also Signale im Bereich von einigen Hertz bis hin zu etwa 20 kHz, wobei in der Praxis üblicherweise ein Frequenzbereich zwischen 50 Hz und 10 kHz ausreichen wird.

Der Begriff Schallwandler erfasst alle Geräte, die aus elektrischen Signalen hörbaren Schall erzeugen, insbesondere also Lautsprecher und Kopfhörer.

Aus sämtlichen der erfassten Datenströme wird gemäß der Erfindung ein kontinuierliches, einheitlich wahrnehmbares akustisches Signal erzeugt, was über die Schallwandler für die überwachte Person selbst als auch für Dritte hörbar sein kann. Um die körperlichen Aktivitäten wahrnehmbar zu machen wird erfindungsgemäß wenigstens ein Parameter des akustischen Signals zeitlich variiert und dabei in Abhängigkeit von aktuellen Datenwerten aus dem ersten Datenstrom bestimmt. Außerdem wird ein Parameter des akustischen Signals zeitlich in Abhängigkeit von aktuellen Datenwerten aus dem zweiten Datenstrom bestimmt und ein Parameter des akustischen Signals wird zeitlich in Abhängigkeit von aktuellen Datenwerten aus dem dritten Datenstrom bestimmt.

Die Parameterbestimmung erfolgt gemäß Rechenvorschriften und/oder gespeicherten Zuordnungen aus den aktuellen Datenwerten. Erfindungsgemäß unterscheiden sich die Rechenvorschriften und/oder gespeicherten Zuordnungen zu den Datenströmen derart paarweise, dass die Veränderung jedes der Datenströme eine zugeordnete hörbare Veränderung des akustischen Signals hervorruft und sich insbesondere die Beeinflussungen durch die unterschiedlichen Datenströme nicht aufheben.

Gemäß der Erfindung wird ein Parameter oder werden mehrere Parameter in unterschiedlicher Weise durch die drei simultan erfassten Datenströme beeinflusst. Einem Hörer bietet sich damit ein akustisches Signal dar, in dem er durch die zeitliche Veränderung des akustischen Signals Rückschlüsse auf die Hirn-Herz- und Atemfunktionen und damit einen Zustand des überwachten Probanden ziehen kann. Das einheitlich dargebotene Signal macht es dem Hörer einfach, intuitiv die unsichtbaren Vorgänge im Organismus des Probanden zu erfassen und wahrzunehmen. Es ist dabei zu beachten, dass die auditive Wahrnehmung des Menschen hinsichtlich ihrer zeitlichen Auflösung und auch der intuitiven Verarbeitung anders entwickelt ist als die visuelle Wahrnehmung. Im Vergleich zum Sehsinn kann das Gehör zwei kurz aufeinanderfolgende Signale relativ gut voneinander unterscheiden, da es im Gegensatz zum Auge keine chemischen Substanzen zerlegen und wieder zusammensetzen muss. Der Mensch kann bis zu 20 Signale pro Sekunde als einzelne Ereignisse, die voneinander getrennt sind, wahrnehmen. Die auditive Wahrnehmung ist in diesem Zusammenhang der visuellen Darstellung überlegen, da visuell beispielsweise Rhythmen mit höheren Frequenzen schlecht eingeschätzt werden können.

Gemäß der Erfindung stehen Variationen und Modulationen für die akustische Wandlung der einzelnen Datenströme zur Verfügung. Lediglich beispielhaft ist an dieser Stelle eine mögliche Wandlung erwähnt, wonach der Datenstrom des EKGs aus dem Herzschlag in einen Parameter des Rhythmus gewandelt wird, der Datenstrom des EEG aus den Gehirnaktivitäten in einen Parameter einer Melodie gewandelt wird und der Datenstrom aus der Atmung einen Parameter eines Obertons bestimmt. Alles zusammen bildet ein Klangbild, bei dem die Werte ineinanderwirken und korreliert werden, um die Interpretierbarkeit der menschlichen Organfunktionen und das Zusammenwirken deutlich zu machen.

Dadurch, dass ein kontinuierliches, einziges akustisches Signal in verschiedenen Parametern verändert wird, ist das Signal deutlich einfacher für einen Hörer zu erfassen, als die bloße Addition oder Überlagerung unabhängiger akustischer Signale.

Die simultane Erfassung und simultane Wandlung sowie simultane Ausgabe der Signalinformationen von den genannten drei wesentlichen Funktionen des Organismus erweitert die akustische Wahrnehmbarkeit des körperlichen und mentalen Zustandes eines überwachten Probanden und ermöglicht eine wesentliche bessere Wahrnehmung von dessen Zustand und seiner körperlichen Aktivität als bisherige Systeme. Ein körperlicher und mentaler Zustand, physiologische Organfunktionen, sowie emotionale und mentale Repräsentationen werden durch die Akustik wahrnehmbar gemacht und deutlich dargestellt. Das Verfahren holt die menschlichen Organfunktionen in die akustische Wahrnehmung der gemessenen Person, sowie der Messanwender.

Alle drei vegetativen Funktionen der Hirnaktivität, des Herzschlags und der Atmung fließen gleichzeitig in eine einzige Klangbearbeitung ein, die es erlaubt, diese zwar simultan aber dennoch in ihrer Bedeutung trennbar voneinander wahrzunehmen.

Das erfindungsgemäße Verfahren und die zugehörige Vorrichtung kann insbesondere eingesetzt werden, um Probanden eine tiefere Einsicht und ein besseres Verständnis in ihre eigene Körperlichkeit und ggf. Erkrankungszustände geben zu können (z.B. für Epileptiker oder Schlaganfallpatienten). Der Einsatz kann als Messinstrument für körperliche oder geistige Funktionen dienen, wie beispielsweise auf der Intensivstation als Überwachungsinstrument, oder als Diagnostikinstrument. Im therapeutischen Einsatz dient die Vorrichtung dem Feedbackverfahren. Individuelle Bio-Feedback-Therapie, beispielsweise Neurofeedback zur Einübung der Selbstwirksamkeit bei Trauma und Trauer oder Krankheiten wie Adipositas, zur Reflexion bei trauenden Menschen und deren Veränderung der physiologischen Strukturen (Herzkreislaufstress und neuronale, eingeschränkte Verarbeitung), zur Rehabilitation nach Schlaganfall oder zum therapeutischen Training bei Multiple Sklerose (MS). Ein bedeutender Einsatz ist das Neurofeedback mit dem zugehörigen Verfahren für Schmerzpatienten in der multimodalen Schmerztherapie, die damit ihren inneren Zustand von Herzkreislauffunktionen und neurologischen Funktionen, bzw. ihrer Gedanken und Gefühle, bzw. ihrer mentalen Repräsentationen prüfen können und lernen, welcher Zustand den Schmerz auf der Schmerzskale reduzieren lässt, oder sogar vergessen lässt. Das Neurofeedback kann ebenfalls eingesetzt werden für Achtsamkeit - gegenüber sich selbst und der Umgebung, allgemein bei der Unterstützung von Schulung der Achtsamkeit, sowie der Wahrnehmungsschulung und Empathieschulung, Reflexion einer "Top Performance" im richtigen Moment, Reflexion der inneren Haltung der Veränderung des menschlichen Organsystems, Optimierte aktive Entspannung, Steigerung des Selbstwertes und der emotionalen Kompetenzen, Optimierung der Fokussierung (Open Focus), bessere Annahme und Verarbeitung von Misserfolgen als Motivationssteigerung.

Außerdem können Ärzte oder Pfleger von einem derartigen System profitieren, da es zum Beispiel als Instrument zur Empathieschulung eingesetzt werden kann. Ihnen kann durch das Verfahren verdeutlicht werden, dass in der Interaktion von zwei oder mehr Menschen eine gegenseitige Wirkung auf das Gegenüber und seine körperlichen und mentalen Funktionen hat. Durch den Einsatz von zwei Messgeräten an zwei Personen, die in einer Interaktion stehen, kann gezeigt werden, dass die Wirkung auf das Gegenüber existiert und die innere Haltung und Wahrnehmungsform einen Einfluss auf das Gegenüber hat. Diese Anwendung hat einen großen Einfluss auf den Umgang mit der Arzt- und-Patientenbeziehung. Durch eine dem Menschen intuitiv vertraute Wahrnehmung durch Klang kann zum Beispiel auch einem Patienten ein Verständnis für die Reaktionen seines Körpers auf unterschiedliche Reize oder in unterschiedlichen Lebenssituationen (z.B. bei Angststörungen) vermittelt werden. Gemäß der Erfindung wird ein umfassendes Bild der organischen Tätigkeit erstellt und gewandelt und in einheitlicher, durch menschliche Sinne leicht verarbeiteter Weise aufbereitet. Diese Anwendung kann neben dem Einsatz für medizinisches, therapeutisches Personal auch für Lehrer in ihrer pädagogischen, zwischenmenschlichen Beziehung zu Schülern erweitert werden oder für Kundenberater, die ebenfalls in einem engen Bezug (Interaktion) zu einem anderen Menschen stehen.

Wie oben beschrieben, bedient sich die Erfindung bei der Erfassung der genannten Daten herkömmlicher Systeme, z.B. einem EEG, einem EKG und einem System zur Erfassung der Atmung, beispielsweise einem Atemgurt. Die von diesen Systemen erfassten eingehenden Signale werden in Digitalsignale gewandelt und aufbereitet. Vor oder nach der Wandlung kann hierzu eine Entfernung von Gleichspannungsanteilen, eine Bandpassfilterung oder Kanalauswahl bei der Erfassung mehrerer Kanäle erfolgen. Außerdem können mehrere Kanäle zusammengemischt und gemittelt werden, oder getrennt weiterverarbeitet werden. Gegebenenfalls sehen die Erfassungsgeräte bereits eine eigenständige A/D-Wandlung der Daten vor, so dass der nachgeschaltete Wandlungsschritt entfallen kann. Die erfassten Datenströme werden gemäß Rechenvorschriften in Verarbeitungsdaten oder Metadaten gewandelt, die zur Klangerzeugung oder Klangmodifikation verwendet werden.

Das erfindungsgemäße Verfahren gliedert sich in daher in die Abschnitte der Signalakquise, der Signalverarbeitung und der Signalausgabe. Gemäß der Erfindung wird im Zuge der Signalverarbeitung eine Zuordnung der aus den akquirierten Signalen abgeleiteten Datenströmen zu Parameteränderungen des Audiosignals vorgenommen. Vor der Ausgabe erfolgt wiederum die Verbindung aller Klangbestimmenden Parameter, so dass bei der Ausgabe das akustische Signal in einer einheitlich wahrnehmbaren Weise dargeboten wird.

Gemäß der Erfindung in ihrer allgemeinen Form können die Auswertungen verschiedener Datenströme gemäß der Parameterbestimmung grundsätzlich denselben Parameter beeinflussen, dort jedoch in unterschiedlicher Weise Einfluss haben. In einer bevorzugten Ausführungsform der Erfindung nehmen die unterschiedlichen Datenströme jedoch jeweils auf andere Paramater des akustischen Signals Einfluss.

Anhand der Beeinflussung verschiedener Parameter des akustischen Signals ist eine intuitive Zuordnung beim Hörer der Signale in einfacher Weise möglich. Beispielsweise kann einer der Datenströme auf den Klang Einfluss haben, ein weiterer Datenstrom hat auf den Pegel bzw. die Lautstärke des akustischen Signals Einfluss und ein weiterer Datenstrom hat auf die Frequenzlage des Schallsignals Einfluss.

Vorzugsweise ist wenigstens einer der Parameter des akustischen Signals ausgewählt aus der Gruppe, bestehend aus dem Schallpegel, der Frequenz und des Spektrums des akustischen Signals.

Beispielsweise kann der Schallpegel des erzeugten akustischen Signals in Abhängigkeit von der Atmung variiert werden, beispielsweise mit der Einatmung ansteigen und bei Ausatmung abnehmen. Außerdem kann beispielsweise die Frequenz des akustischen Signals durch einen anderen Datenstrom, beispielsweise die aus dem EEG abgeleiteten Datenströme variiert werden. Dazu können zum Beispiel die erfassten Spannungssignale aus der Hirnaktivität in den hörbaren Bereich transformiert werden. Das Spektrum des akustischen Signals und die damit verbundene Klangfarbe kann ebenfalls in Abhängigkeit eines der Datenströme, zum Beispiel in Abhängigkeit von der Herzfrequenz variiert werden, so dass das akustische Signal ein mit der Herzfrequenz veränderliche und pulsierende Klangfarbe erhält.

Durch eine solche Zuordnung sind sämtliche Ereignisse zeitgleich, jedoch weiterhin trennbar zu erfassen, da der Hörer die wahrnehmbaren Rhythmen und Variationen der Parameter in Zuordnung zu diesen Rhythmen intuitiv den zugehören Körperfunktionen zugeordnet.

Gemäß der Erfindung ist es grundsätzlich beliebig, auf welcher Weise ein Signal aus den Datenströmen erzeugt wird. Entsprechend kann also zum Beispiel durch Transformation des Datenstromes aus den Messungen der Hirnaktivität durch Transformation in den hörbaren Frequenzbereich ein Signal errechnet werden, dessen Klangfarbe bzw. Spektrum und Lautheit bzw. Schaldruckpegel durch die anderen Datenströme beeinflusst wird. Die vorliegende Vorrichtung stellt modular klangliche Möglichkeiten zur Darstellung der Parameter organischer Funktionen und deren Signale zur Verfügung.

In einer bevorzugten Ausgestaltung der Erfindung wird jedoch das akustische Signal erzeugt, indem ein akustisches Basissignal wenigstens eine akustische Filterung durchläuft, wobei die Parameter der wenigstens einen akustischen Filterung in Abhängigkeit von den aktuellen Werten wenigstens eines der Datenströme variiert werden.

In dieser Ausgestaltung wird also ein Filterparameter in Abhängigkeit von einem der Datenströme gewählt, der ein vorhandenes Basissignal in Abhängigkeit von den aktuellen Werten des Datenstromes verändert. Es können auch eine Mehrzahl von Filter durchlaufen werden, die jeweils in einem oder mehreren Parametern durch einen oder mehrere der Datenströme beeinflusst werden.

Das akustische Basissignal kann dabei in Abhängigkeit von einem oder mehreren der Datenströme kreiert oder daraus abgeleitet werden, bevor es die Filterung durchläuft.

In einer bevorzugten Ausgestaltung der vorangehenden Ausführungsform umfasst die akustische Filterung wenigstens ein Resonanzfilter oder ein Formantfilter. Resonanzfilter werden allgemein verwendet, um eine parametrisierte Frequenz auf einen bestimmten Pegel zu verstärken oder abzudämpfen. Ein in Abhängigkeit von einem Datenstrom variierbarer Resonanzfilter kann also beispielsweise vorsehen, dass die gewählte Resonanzfrequenz, die zugehörige Bandbreite des Filters oder dessen Verstärkung in Abhängigkeit von aktuellen Werten des Datenstroms variiert wird.

Ein Formantfilter betont mehrere Frequenzen, insbesondere Frequenzen aus dem Lautspektrum der menschlichen Sprache. In Abhängigkeit der Parameter eines Formantfilters werden die verstärkten und unterdrückten Frequenzen verändert. Zur Konfiguration derartiger Filter können Formanttabellen verwendet werden, die allgemein verfügbar sind und für eine Vielzahl von menschlichen Lautäußerungen, insbesondere Vokalerzeugungen der menschlichen Stimme, Gruppen von zugehörigen Formantfrequenzen aufweist. Die tabellarische Auswahl und Filterparametrisierung kann in Abhängigkeit von einem oder mehreren der Datenströme erfolgen.

Vorzugsweise wird ein Pegel des akustischen Signals zeitlich in Abhängigkeit von aktuellen Daten aus dem zweiten oder dritten Datenstrom variiert. Der zweite Datenstrom enthält Informationen zu Signalen, die aus der Herzaktivität des menschlichen Organismus abgeleitet sind. Der dritte Datenstrom enthält Informationen über den zeitlichen Signalverlauf aus Messungen der Atmung des menschlichen Organismus. Die zeitliche Variation des Signalpegels, beispielsweise eine Pegeländerung im Rhythmus der Herzfrequenz oder Atemfrequenz gibt dem Hörer des akustischen Signals eine intuitive und unmittelbare Vermittlung des diesbezüglichen körperlichen Rhythmus am Probanden. Es ist dabei allerdings nicht zwingend erforderlich, dass der Pegel sich mit derselben Frequenz ändert wie die zugrundeliegende körperliche Frequenz. Vielmehr ist es ohne weiteres möglich, aus dem Datenstrom zugehörige Daten abzuleiten und beispielsweise eine Herzfrequenz oder Atmungsfrequenz in einen Lautstärkepegel zu transformieren, so dass bei Beschleunigung der Herzfrequenz eine die Herzschlagperioden übergreifende Pegelsteigerung im akustischen Signal resultiert. Eine Verminderung der Herzfrequenz und Beruhigung des Probanden geht dann mit einer Gesamtpegelabnahme des Signals einher. Für die Pegelermittlung können beispielsweise Frequenzbestimmungen über einen Zeitraum von mehreren Sekunden, beispielsweise 5 bis 10 Sekunden herangezogen werden.

In einer weiteren Ausführungsform der Erfindung wird als Basissignal ein synthetisch erzeugtes Basissignal verwendet. Unter synthetisch erzeugten Klängen sind allgemein Klänge zu verstehen, die mit Hilfe eines elektrischen Gerätes zur Klangerzeugung erzeugt werden. Insbesondere sind damit Klänge gemeint, die mit einem Synthesizer erzeugt werden (Granularsynthese). Die eigentliche Erzeugung des akustischen Basissignals mit einem Synthesizer kann dabei sowohl in Abhängigkeit als auch gänzlich unabhängig von den Datenströmen erfolgen. Beispielsweise kann eine Grundklangfolge oder ein Rauschen erzeugt werden, welchem erst durch die Filterung in Abhängigkeit von den Datenströmen eine Charakteristik gemäß den organischen Funktionen des menschlichen Körpers aufgeprägt wird. Alternativ kann auch das Basissignal synthetisch in Abhängigkeit von den aktuellen Daten wenigstens eines der Datenströme erzeugt werden, beispielsweise indem die Daten der Hirnaktivität in das hörbare Spektrum transformiert werden und als Rauschsignal als erzeugtes synthetisches Basissignal verwendet werden. Ebenso können Klänge zur akustischen Darstellung der Signale eingesetzt werden, die als Klangsynthese aus eingespielten, aufzeichneten Klängen oder mitgeschnittenen Musikaufzeichnungen entwickelt werden. Es könnten Musikstücke eingespielt werden und in dem System transformiert und verwendet werden.

In einer alternativen Gestaltung der Erfindung ist das Basissignal aus einer gespeicherten Folge von Schallereignissen erzeugt, insbesondere aus einer gespeicherten Klangfolge oder Melodie.

Diese alternative Gestaltung sieht vor, vorbereitete und gespeicherte Klangdaten, also Samples, als Basis zu verwenden und diesen durch Filterung die Veränderungen gemäß den erfassten Datenströmen aufzuprägen. Alternativ kann eine Melodie auch als Notendaten vorliegen oder zufällig erzeugt werden. Die Eigenschaften der Schallaufzeichnungen können so gewählt werden, dass die aufgeprägten Charakteristika eindeutig identifizierbar sind. Dies könnte den Rhythmus betreffen, den Oberton oder auch die Melodie. Die Melodie aus dem EEG könnte beispielsweise aus eingespielten Mönchschorälen oder eingespielten Geigensequenzen entwickelt werden oder auf Grundlage von Granularsynthese aufgebaut sein.

In einer besonderen Gestaltung dieser Ausführungsform mit gespeicherten Schallereignissen als Basissignal werden diese gespeicherten Schallereignisse in Abhängigkeit von den aktuellen Datenwerten wenigstens eines der Datenströme ausgewählt. Beispielsweise können für verschiedene Atemfrequenzen oder Herzfrequenzen jeweils Basissignale als gespeicherte Schallereignisse vorliegen, die gemäß einer vorgegebenen Zuordnung ausgewählt werden. Beispielsweise kann für einen bestimmten Herzfrequenzkorridor eine zugehörige abgespeicherte Tonfolge verwendet werden, wohingegen bei steigender Herzfrequenz alternative Melodien gewählt werden. In jedem Fall wird das Basissignal anschließend in Abhängigkeit von den übrigen Datenströmen gefiltert und variiert. So kann zum Beispiel die Art der Melodie oder sonstigen Klangfolgen Auskunft über die Hirnaktivität oder Herzaktivität geben, während deren Verzerrung und Klangfarbe sowie Lautstärkeauskunft über die übrigen überwachten Körperfunktionen geben. Das System ist modular entwickelt und kann flexibel geschaltet werden, um die geeignete klangliche Darstellung der gemessenen Situation wiederzugeben.

Eine weitere alternative Gestaltung sieht vor, eine zur Laufzeit erzeugte Tonfolge bzw. Melodie für einen Teil der Datenströme zu verwenden und Eigenschaften dieser Tonfolge gemäß den erfassten Datenströmen zu verändern. Eine Tonfolge kann eine aus Zufallstönen erzeugte Melodie sein, die durch einen Synthesizer abgespielt wird oder wo wiederum aufgezeichnete Schallereignisse abgespielt werden. Neben den beschriebenen Möglichkeiten der Filterung zur Veränderung des Klangs können Parameter der Tonfolge sein: Tonhöhe, Tonlänge, Tonumfang, Auswahl einer musikalischen Skala, Rhythmus, Tempo, Rhythmusvariation. Diese so erzeugte Melodie macht ebenso wie die anderen Gestaltung alle Eingangs-Datenströme identifizierbar und unterscheidbar.

Neben den oben vorgestellten erfinderischen Verfahren und dessen Ausgestaltungen wird außerdem eine Vorrichtung zur Durchführung jedes der Verfahren offenbart. Dazu weist die erfindungsgemäße Vorrichtung wenigstens drei Schnittstellen auf, um zeitliche Signalverläufe aus Messungen der Aktivitäten des Gehirns, des Herzens und der Atmung aufzunehmen. Diese Schnittstellen können so ausgebildet sein, dass sie für die Rohdaten von gängigen medizinischen Instrumenten kompatibel sind, die diese Rohdaten als Ausgangssignale zur Verfügung stellen. Entscheidend ist, dass die Daten der Messgeräte live in das System gespielt werden und nicht erst als Archivdaten, d.h. die Rohdaten werden als Ausgangssignale direkt zur Verfügung gestellt. Die erfindungsgemäße Vorrichtung weist wenigstens eine Wandlereinrichtung auf, die mit den Schnittstellen gekoppelt ist und die zeitlichen Signalverläufe in wenigstens drei Datenströme wandelt. Eine Steuer- und Auswerteeinrichtung ist mit der Wandlereinrichtung gekoppelt, um die digitalisierten Datenströme aufzunehmen und zu verarbeiten. Eine Verarbeitung kann eine Filterung, Wandlung, Kombination oder sonstige Rechenverarbeitung umfassen. Die Steuer- und Auswerteeinrichtung kann insbesondere durch einen Computer mit zugehöriger Verarbeitungssoftware gebildet sein.

Mit der Steuer- und Auswerteeinrichtung ist eine Klangerzeugungseinrichtung gekoppelt, die wiederum von der Steuer- und Auswerteeinrichtung angesteuert wird. Die Klangerzeugungseinrichtung kann einen Synthesizer und eine Mehrzahl von Filtern umfassen, welche von der Steuer- und Auswerteeinrichtung in Abhängigkeit von den aktuellen Werten in den Datenströmen parametrisiert werden. Die Klangerzeugungseinrichtung weist eine Schnittstelle auf, die zum Anschluss wenigstens einem Schallwandler dient. Entsprechend ist die Klangerzeugungseinrichtung beispielsweise mit einem Kopfhöreranschluss oder einem Lautsprecheranschluss ausgestattet.

Im Rahmen der Erfindung ist es möglich, neben den genannten Datenströmen, die aus den elektrischen Aktivitäten des Hirns, den elektrischen Aktivitäten des Herzens und aus der Atmung abgeleitet werden, weitere Körperdaten in Datenströme zu wandeln und zusätzlich zur Modifikation des akustischen Signals einzusetzen. Beispielsweise kann eine Messung des Hautwiderstandes verwendet werden, um einen weiteren Datenstrom zu generieren, der die Klangerzeugung des akustischen Signals beeinflusst.

Das erfindungsgemäße Verfahren und die zugehörige Vorrichtung kann insbesondere eingesetzt werden, um zwei Personen und deren Wechselbeziehung bei einer Interaktion mit mehr oder weniger Empathie - einem aufeinander eingehen - zu messen, bzw. die Harmonisierung der Messwerte darzustellen, indem die Annäherung organischen Funktionen der beiden gemessenen Personen auch in den Klängen zu beobachten ist. Die Klänge bilden zusammen ein neue Klangsynthese.

Außerdem ist es möglich, eine Mehrzahl der Verfahren mit mehreren Probanden gleichzeitig durchzuführen. Dabei können die Probanden zum Beispiel die erzeugten akustischen Signale aus den körperlichen Aktivitäten eines anderen Probanden hören. Es hat sich gezeigt, dass auf diese Weise eine gewisse Harmonisierung und Angleichung von körperlichen Zuständen zwischen mehreren Personen erreichbar ist. (siehe auch "Brains Swinging in Concert: Cortical Phase Synchronization While Playing Guitar", Ulman Lindenberger, Shu-Chen Li, Walter Gruber and Viktor Müller BMC Neuroscience 10:22,17 March 2009)

Zusätzlich zu der oben beschriebenen Erfindung können ergänzende Daten des menschlichen Organismus erfasst werden, um das Zusammenspiel der vegetativen Herzkreislauffunktionen und der kognitiven Leistungen zeitgleich zu erfassen und darzustellen. Dazu gehört z.B. eine Messung der Sauerstoffsättigung per Fingerclip für Pulsoximeter, die periphere Fingerpulskurve, sowie die Pulswellengeschwindigkeit. Als Analysewert kann zusätzlich eine Herzfrequenzvariabilität im System als Datenstrom integriert.

Das erfindungsgemäße Verfahren der Akustik kann ergänzt werden durch eine Umsetzung der kombinierten Parameter in eine visuelle Darstellung. Statt der der akustischen Umsetzung in Hörwellen entsteht ein Bild, indem die Farben den Messwerten/Parametern zugeordnet werden. Die Höhe der Messwerte wird in Farbintensitäten dargestellt. Somit entsteht zusätzlich zum akustischen Klangbild ein visuelles Bild der Vorgänge im menschlichen Organismus.

Die visuelle Darstellung wird als Ergänzung zur auditiven Darstellung genutzt, um die Interpretation zu verdeutlichen. Das Zusammenspiel der Parameter wird aber durch die akustische Wandlung deutlich und das Farbbild zeigt parallel einen Zustand.

Die Erfindung wird nun anhand der beiliegenden Zeichnungen näher erläutert.
Figur 1 zeigt schematisch eine Vorrichtung zur Anwendung des erfindungsgemäßen Verfahrens gemäß einem ersten Ausführungsbeispiel;
Figur 2 zeigt eine Blockdarstellung der Verarbeitungseinrichtung aus Figur 1;
Figur 3 zeigt aus schematisch die Verwendung des erfindungsgemäßen Verfahren bei der Interaktion zwei Menschen;

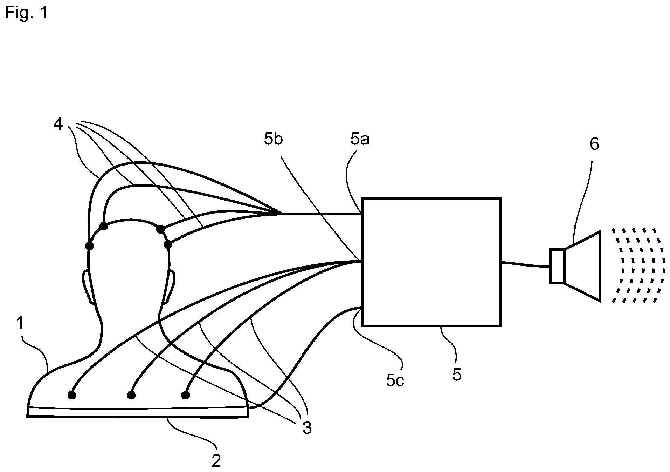

Figur 1 zeigt einen Probanden 1, der mit einer Mehrzahl an Erfassungsgeräten gekoppelt ist. Der Proband 1 trägt ein Brustgurt 2, welcher die Atmung des Probanden 1 erfasst. Ein EKG 3 ist ebenfalls mit dem Probanden gekoppelt. Zur Erfassung der Hirnaktivität ist ein EEG 4 über mehrere Signalabnehmer mit dem Kopf des Probanden 1 gekoppelt. Bei den zugehörigen Geräten handelt es sich um Geräte gemäß dem Stand der Technik, die in dieser Darstellung nicht gezeigt sind, da ihre Ausgestaltung nicht wesentlich für die Erfindung ist. Wesentlich ist, dass sie mit einer Verarbeitungseinrichtung 5 gekoppelt sind, welche Schnittstellen 5a, 5b, 5c für die Erfassungsgeräte aufweist. Die Verarbeitungseinrichtung kann ein Computersystem umfassen.

Eine Wandlung der als Analogsignale erfassten Signale kann entweder in den Erfassungsgeräten selbst, also innerhalb des EEG, des EKG und des Brustgurtes erfolgen, so dass über die Schnittstellen 5a, 5b, 5c bereits digitale Signale übertragen werden. Alternativ kann eine Analog-Digitalwandlung an den Schnittstellen der Verarbeitungseinrichtung 5 erfolgen. Mit der Verarbeitungseinrichtung 5 ist ein Schallwandler 6 in Gestalt eines Lautsprechers gekoppelt, der beispielsweise an eine Soundkarte des Computersystems der Verarbeitungseinrichtung 5 angeschlossen ist. Das Bild der Figur 1 zeigt damit die wesentlichen Komponenten des erfindungsgemäßen Systems, wobei dargestellt ist, dass die Erfassung einer Mehrzahl von komplexen und rasch veränderlichen Informationen an einen Probanden 1 in ein einheitliches hörbares akustisches Signal gewandelt wird, welches über den Schallwandler 6 ausgegeben wird. Dieses akustische Signal wird durch die sämtlichen erfassten Daten und daraus abgeleiteten Datenströme beeinflusst. Es ist unmittelbar ersichtlich, dass eine intuitive Erfassung des akustischen Signals nach Ausgabe möglich ist, während die analytische In-Situ-Beobachtung der erfassten Werte der Körperfunktionen des Probanden 1 kaum möglich ist.

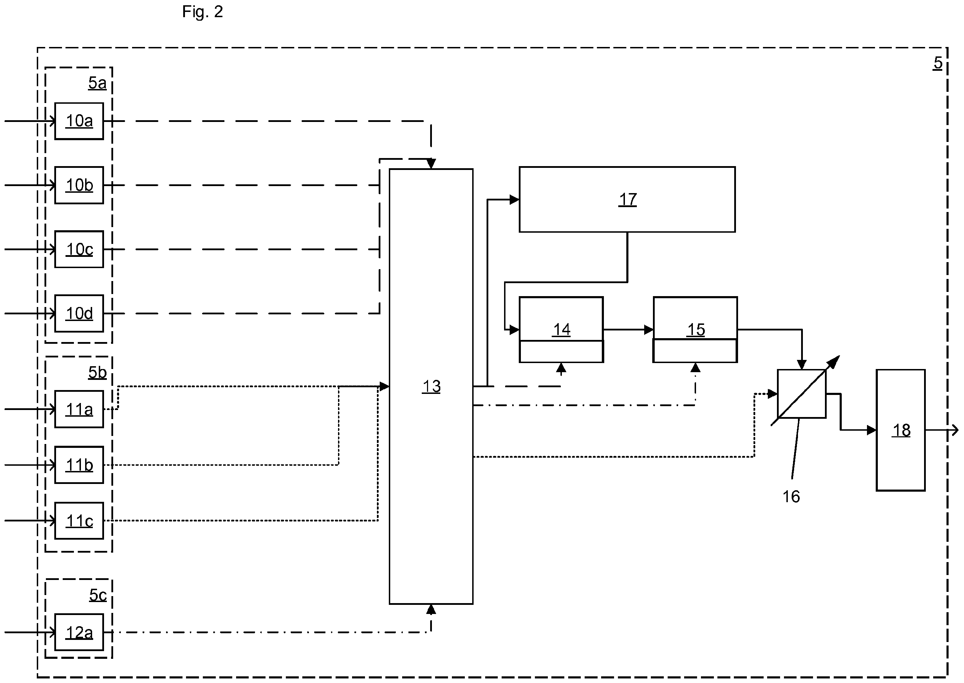

Figur 2 zeigt eine Blockdarstellung der Verarbeitungseinrichtung der Erfindung. In diesem Beispiel ist die Verarbeitungseinrichtung so ausgebildet, dass sie mit der Filterung von synthetisch erzeugten Signalen arbeitet. Auf der linken Seite der Zeichnung sind Signaleingänge 10a, 10b, 10c, 10b gezeigt, welche in diesem Beispiel Schnittstellen für den Eingang der Signale eines EEG darstellen. Die Schnittstellen 10a, 10b, 10c, 10d können in der Praxis als einheitliche Schnittstelle ausgebildet sein. Die Schnittstellen nehmen die Signalverläufe des EEG auf und wandeln diese in digitale Datenströme, die sie eine zentrale Verarbeitungseinheit 13 weiterleiten.

Die weiteren Schnittstellen 11a, 11b, 11c nehmen Signalverläufe aus einem EKG auf und wandeln diese ebenfalls in digitale Datenströme, die weitergeleitet werden an eine zentrale Verarbeitungseinheit 13.

Die Signale eines Atemgurtes werden über die Schnittstelle 12a gewandelt und der zentralen Verarbeitungseinheit 13 zugeführt.

Die zentrale Verarbeitungseinheit 13 kann durch ein entsprechend konfiguriertes und programmiertes Computersystem gebildet sein. In der zentralen Verarbeitungseinheit 13 laufen entsprechend simultan fortlaufend Daten ein, die an einen Probanden bezüglich seiner Hirnaktivität, Herzaktivität und Atmung erfasst werden. Mit der zentralen Verarbeitungseinheit 13 gekoppelt sind ein Resonanzfilter 14 sowie ein Formantfilter 15 und eine Pegelsteuerung 16. Ein Synthesizer 17 ist in diesem Beispiel als Klangerzeugungseinheit vorgesehen. Der Synthesizer 17 erzeugt ein Signal, welches von der zentralen Verarbeitungseinheit 13 hinsichtlich seiner Parameter beeinflusst wird. Im Synthesizer kann zur Klangsignalerzeugung beispielsweise eine Kombination von Rauschsignalen und Wellenformen verwendet werden, deren Parameter (zum Beispiel Frequenzen und Amplituden) in Abhängigkeit von den eingehenden Datenströmen variiert werden. Damit kann beispielsweise die Tonhöhe, die Auswahl der Wellenformen in Abhängigkeit von den jeweils gemessenen Körperfunktionen erzeugt werden.

Das aus dem Synthesizer 17 hervorgebrachte Signal durchläuft das Resonanzfilter 14, dessen Parameter, wie oben bereits beschrieben, in Abhängigkeit von den Datenströmen beeinflusst wird. In diesem Beispiel wird das Resonanzfilter in Abhängigkeit ausschließlich der Datenströme bezüglich der Hirnaktivität parametrisiert.

Anschließend durchläuft das Signal ein Formantfilter, dessen Filterfrequenzen in diesem Ausführungsbeispiel in Abhängigkeit von den erfassten Datenströmen aus der überwachten Atmung des Probanden abgeleitet werden.

Schließlich durchläuft das Signal eine Pegelanpassung 16, die in Abhängigkeit von den erfassten Herzdaten angesteuert wird. Der Pegel kann sich also entsprechend des jeweiligen Herzrhythmus ändern oder auch anhand anderer Herzdaten, beispielsweise der Herzfrequenz über periodenübergreifende Dauern geändert werden.

Das resultierende Signal wird anschließend im Aufbereiter 18 für die Signaldarbietung aufbereitet und dann als Signal, insbesondere Stereosignal ausgegeben. Dies kann beispielsweise über handelsübliche Soundkarten und mittels angeschlossenem Kopfhörer oder über den in Fig. 1 gezeigten Lautsprecher 6 erfolgen.

Die verschiedenen Biosignale (EEG, EKH, Atmung) werden damit im ausgegebenen Klang oder Audiosignal sowohl zeitlich als auch spektral so dargeboten, dass sie akustisch gut trennbar und damit einzeln wahrnehmbar sind. Der Hörer eines solchen Signals kann entweder nach kurzer Einführung oder intuitiv aus den Klangbestandteilen auf die zugehörigen Biosignale des Körpers Rückschlüsse ziehen, da die Signale hinsichtlich ihrer Rhythmen intuitiv erkennbar sein können.

Es ist möglich, die Signale von mehreren Probanden zu einem einheitlichen Signal aufzubereiten. Dabei kann beispielsweise für jede Person eine andere Tonhöhe, ein anderer Klang oder auch eine andere Raumposition bei der Aufbereitung durch den Aufbereiter 18 im Stereosignal gewählt werden.

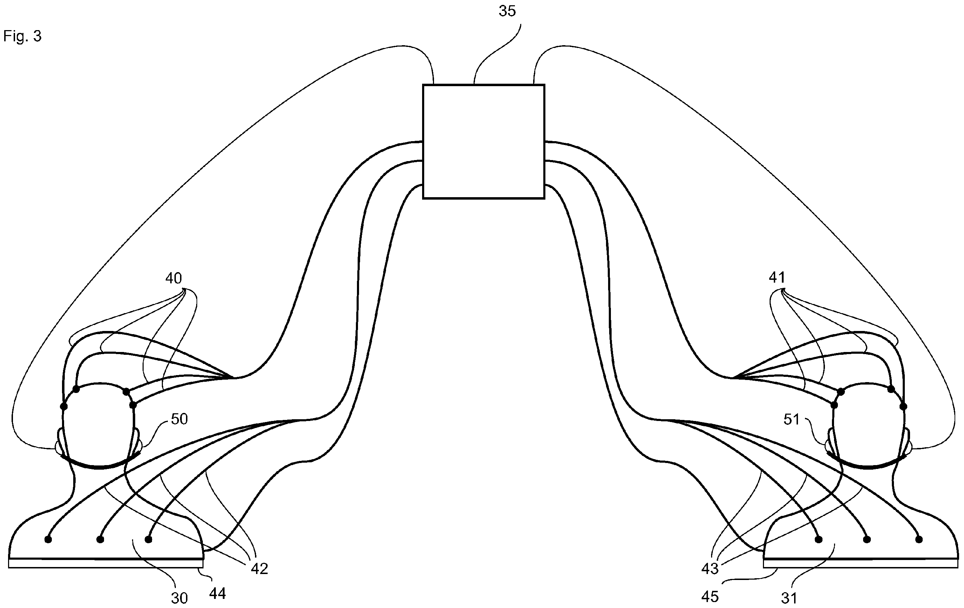

In Figur 3 ist eine weitere Ausführungsform der Erfindung dargestellt. Hierbei wird der Aspekt der Interaktion zwischen zwei Personen 30, 31 technisch umgesetzt. Die erfindungsgemäße Vorrichtung 35 wird für beide Personen 30, 31 verwendet während die Personen interagieren.

Erneut werden von jeder der Personen 30, 31 die Hirnaktivität mit Signalabnehmern 40; 41 überwacht. Die Herzsignale werden mit Signalabnehmern 42; 43 abgenommen und die Atmungsüberwachung erfolgt mit jeweils einem Brustgurt 44, 45.

In diesem Ausführungsbeispiel werden Schallereignisse in Abhängigkeit von den aktuellen Datenwerten von zwei Personen gleichzeitig dargestellt. Das modulare System erlaubt eine Zuordnung aller Eingangsdatenströme zu den beschriebenen Parametern, sodass sowohl alle erfassten Daten als auch die Daten der einzelnen Person erkennbar und unterscheidbar sind.

Daneben ist es möglich, unter klangästhetischen Gesichtspunkten ein gutes Zusammenspiel aller Schallereignisse zu erreichen. Die Module lassen ein Audiosignal zu, also Samples, wie z.B. eine Basedrum für das Herz, die in dem Zusammenspiel bei der zweiten Vorrichtung zu einer dazu passenden Snare-Drum definiert, bzw. formiert wird. Zusammen klingen diese beiden klanglichen Umsetzungen miteinander und drücken den selben Parameter aus, der auf die anderen Datenströme des Weiteren in beiden Vorrichtungen einwirkt. Es kann aber auch eine Notenfolge sein, die wiederum abhängig von den Parametern zufällig erzeugt wird. Die Verklanglichung von zwei gleichzeitig laufenden Klangwandlungen erfordert eine Auswahl von zwei passenden Notenfolgen, die in Stimmung und Tonhöhe passen. Was in einem Monolog einer Person umgesetzt wird, entwickelt sich in diesem Teil des Verfahren zu einem Dialog von zwei Systemen. Gemäß dem erfindungsgemäßen Verfahren werden biologische Aktivitäten von zwei menschlichen Organismen in Schallinformationen gewandelt. Dazu wird zumindest ein erster zeitlicher Signalverlauf aus Messungen einer elektrischen Aktivität von zwei Gehirnen (EEG), der Herzaktivität (EKG), sowie der Atmung des jeweils menschlichen Organismus erzeugt. Auch in dieser weiteren Ausführung der Erfindung werden von den zwei untersuchten Probanden selbst wie auch für ungeschulte Beobachter die Interpretierbarkeit der Signale möglich.

Die Ausgabe der Klangsignale erfolgt an die Personen 30, 31 über Kopfhörer 50; 51. Dabei kann die erfindungsgemäße Vorrichtung 35 die Signale beider Personen gemeinsam ausgeben, so dass beide Personen 30, 31 dieselbe Klanginformation dargeboten bekommen. Es ist jedoch auch möglich, jeder Person die aus den eigenen Körpersignalen gewandelte Klanginformation zu übermitteln.

Schließlich ist es ebenfalls möglich, jeder der Personen 30, 31 die Klanginformation aus den Körpersignalen der jeweils anderen Person darzubieten. Die ist insbesondere bei Therapien oder Empathieschulungen hilfreich.

## Patentansprüche

1. Verfahren zum Generieren von Schallinformationen in Abhängigkeit von biologischen Aktivitäten eines menschlichen Organismus,
wobei zumindest ein erster zeitlicher Signalverlauf aus Messungen einer elektrischen Aktivität eines Gehirns des menschlichen Organismus erzeugt wird, wobei aus dem ersten zeitlichen Signalverlauf ein erster Datenstrom generiert wird,
wobei zumindest ein zweiter zeitlicher Signalverlauf aus Messungen einer elektrischen Aktivität eines Herzens des menschlichen Organismus erzeugt wird, wobei aus dem zweiten zeitlichen Signalverlauf ein zweiter Datenstrom generiert wird,
wobei zumindest ein dritter zeitlicher Signalverlauf aus Messungen einer Atmung des menschlichen Organismus erzeugt wird, wobei aus dem dritten zeitlichen Signalverlauf ein dritter Datenstrom generiert wird,
wobei in Echtzeit ein akustisches Signal im auditiv wahrnehmbaren Frequenzspektrum erzeugt und über Schallwandler ausgegeben wird,
wobei wenigstens ein Parameter des akustischen Signals zeitlich in Abhängigkeit von aktuellen Datenwerten aus dem ersten Datenstrom bestimmt wird,
wobei wenigstens ein Parameter des akustischen Signals zeitlich in Abhängigkeit von aktuellen Datenwerten aus dem zweiten Datenstrom bestimmt wird,
wobei wenigstens ein Parameter des akustischen Signals zeitlich in Abhängigkeit von aktuellen Datenwerten aus dem dritten Datenstrom bestimmt wird,
wobei die Parameterbestimmung gemäß Rechenvorschriften und/oder gespeicherten Zuordnungen aus den Datenwerten abgeleitet wird,
wobei sich die Rechenvorschriften und/oder gespeicherten Zuordnungen zu den Datenströmen derart paarweise unterscheiden, so dass die Veränderung jedes der Datenströme eine zugeordnete hörbare Veränderung des akustischen Signals hervorruft.

2. Verfahren nach Anspruch 1, wobei jeder der Datenströme einen anderen Parameter des akustischen Signals beeinflusst.

3. Verfahren nach einem der vorangehenden Ansprüche, wobei wenigstens einer der Parameter des akustischen Signals ausgewählt ist aus der Gruppe, bestehend aus dem Schallpegel, der Frequenz, des Spektrums.

4. Verfahren nach einem der vorangehenden Ansprüche, wobei das akustische Signal erzeugt wird, indem ein akustisches Basissignal wenigstens eine akustische Filterung durchläuft, wobei die Parameter der wenigstens einen akustischen Filterung in Abhängigkeit von den aktuellen Werten wenigstens eines der Datenströme variiert werden.

5. Verfahren nach Anspruch 4, wobei die akustische Filterung ein Resonanzfilter oder ein Formantfilter umfasst und wenigstens ein Parameter des Resonanzfilters oder Formantfilters zeitlich in Abhängigkeit von aktuellen Daten aus einem der Datenströme variiert wird.

6. Verfahren nach einem der Ansprüche 3 bis 5, wobei der Pegel des akustischen Signals zeitlich in Abhängigkeit von aktuellen Daten aus dem zweiten oder dritten Datenstrom variiert wird.

7. Verfahren nach einem der Ansprüche 4 bis 6, wobei das akustische Basissignal ein synthetisch erzeugtes Basissignal ist.

8. Verfahren nach Anspruch 7, wobei das Basissignal synthetisch in Abhängigkeit von den aktuellen Daten wenigstens eines Datenstromes erzeugt wird.

9. Verfahren nach einem der Ansprüche 4 bis 6, wobei das Basissignal aus einer gespeicherten Folge von Schallereignissen erzeugt wird, insbesondere aus einer gespeicherten Klangfolge oder Melodie.

10. Verfahren nach Anspruch 9, wobei die gespeicherte Folge von Schallereignissen in Abhängigkeit von den aktuellen Datenwerten wenigstens eines der Datenströme ausgewählt wird.

11. Vorrichtung zur Durchführung eines Verfahrens gemäß einem der Ansprüche 1 bis 10,
mit wenigstens drei Schnittstellen (5a, 5b, 5c),
wobei eine erste Schnittstelle (5a) als Eingang für den ersten zeitlichen Datenstrom aus Messungen einer elektrischen Aktivität eines Gehirns des menschlichen Organismus ausgebildet ist,
wobei eine zweite Schnittstelle (5b) als Eingang für den zweiten zeitlichen Datenstrom aus Messungen einer elektrischen Aktivität eines Herzens des menschlichen Organismus ausgebildet ist,
wobei eine dritte Schnittstelle (5c) als Eingang für den dritten zeitlichen Datenstrom aus Messungen einer Atmung des menschlichen Organismus ausgebildet ist,
wobei eine Steuer- und Auswerteeinrichtung (13) mit den Schnittstellen (5a, 5b, 5c) gekoppelt ist,
wobei eine Klangerzeugungseinrichtung (17, 14, 15, 16, 18) mit der Steuer- und Auswerteeinrichtung gekoppelt ist,
wobei die Steuer- und Auswerteeinrichtung (13) zur Ansteuerung der Klangerzeugungseinrichtung (17, 14, 15, 16, 18) in Abhängigkeit von den Datenströmen ausgebildet ist
wobei die Klangerzeugungseinrichtung (17, 14, 15, 16, 18) eine Schnittstelle zum Anschluss von wenigstens einem Schallwandler (6) aufweist.

12. Vorrichtung nach Anspruch 11, wobei den Schnittstellen jeweils wenigstens eine Wandlereinrichtung vorgeschaltet ist, welche die zeitlichen Signalverläufe jeweils in Datenströme wandelt.
